# EUROPEAN PATENT SPECIFICATION

(11) **EP 1 267 706 B1**
(45) Date of publication and mention of the grant of the patent: **01.10.2003**
(21) Application number: 01914160.5
(22) Date of filing: 20.03.2001
(51) Int. Cl.: A61B 5/00, A61B 5/0404, A61B 5/0408

(54) **PORTABLE ECG SIGNALING DEVICE**
TRAGBARE VORRICHTUNG ZUR EKG-SIGNALISIERUNG
DISPOSITIF DE SIGNALISATION D'ECG PORTATIF

(30) Priority: 23.03.2000 IL 13524000
(43) Date of publication of application: 02.01.2003
(73) Proprietor: SHL Telemedicine International Ltd., 67891 Tel Aviv (IL)
(72) Inventor: ALROY, Yoram, 67891 Tel Aviv (IL); REINHOLD, Herbert, Arnold, MD 21012 (US)
(74) Representative: Grünecker, Kinkeldey, Stockmair & Schwanhäusser Anwaltssozietät
(86) International application number: IL0100261
(87) International publication number: WO01070101

(56) References cited:
- EP-A- 0 657 136
- WO-A-94/03106
- WO-A-97/19631
- US-A- 4 889 134
- US-A- 4 958 645
- US-A- 5 919 141

## Description

### FIELD OF THE INVENTION

This invention relates to a portable ECG signaling device.

### BACKGROUND OF THE INVENTION

Patients having a history of medical ailments not infrequently subscribe to a medical monitoring service on an ambulatory basis. Upon effecting communication with a monitoring unit, the patient is frequently required to undertake an interactive dialog with medical personnel at the monitoring unit so as to enable the medical personnel to diagnose the patient's medical symptoms. Since many of those who are particularly at risk suffer from heart-disease, an ECG is usually one of the first tests which should be carried out. To this end, much effort has been directed to the provision of portable instruments for allowing a patient to carry out an ECG on himself. At their most rudimentary, such instruments comprises a pair of electrodes which are held against a patient's body, usually near his chest to detect an electrical voltage indicative of the electrical activity of the heart. The resulting current waveform response permits partial determination of the patient's cardiac health. A more detailed determination may be realized by using more than two electrodes and portable devices are known having, for example, ten electrodes mounted on a common carrier and amenable to placement on a patient's chest area by the patient with minimum effort.

US Patent No. 5,339,823 (Reinhold, Jr.) shows such a device for obtaining electrical heart activity of an individual in a form capable of producing a twelve-lead electrocardiogram of an individual. The device includes a portable electrode support having an array of six non-adhesive precordial electrodes fixed thereon at predetermined positions within the array which correspond with the Wilson precordial leads for the individual. The device also includes a right arm electrode, a left arm electrode, a left leg electrode and circuitry for converting the electrical heart activity of the individual obtained by said electrodes into a form capable of producing a twelve-lead electrocardiogram. In use, the left leg, left arm and right arm electrodes are applied to the skin of the individual at locations such that the circuitry can be electrically operable to obtain leads I, II, III, AVR, AVL, and AVF therefrom. Human pressure is applied to engage the array of six precordial electrodes with the skin of the chest of the individual in an operative relation, and circuitry is operated for a time sufficient to obtain electrical heart activity of the individual in a form capable of producing an electrocardiogram.

In such a device, it takes a certain amount of time for the complete ECG data to be measured and transmitted from the patient to the remote monitoring center, and during this time the patient must hold the ECG monitor securely in place. Typically, for a twelve lead ECG, data is fed serially from each electrode and the time taken for a complete set of measurements to be read and transmitted is approximately one minute. Of this, some 20 seconds are required to transmit the patient's rhythm strip which is detected using two electrodes only and gives an indication as to whether the patient's heart beat is uneven. This is followed by the serial transmission of data from each of the remaining electrodes for a duration of approximately 45 seconds. This is along time for the patient to hold the device steady, particularly bearing in mind that patients requiring ECG monitors are by definition infirm and often elderly and unsteady.

US Patent No. 5,365,935 (Righter *et al*.) discloses a portable, multi-channel ECG data monitor/recorder having electronic circuitry for selectively monitoring and recording a patient's electrocardiographic (ECG) data signals. A first electrode is disposed on a first surface of the apparatus casing for contact with an ECG lead position on the patient's body and second and third paste-on electrodes are attached on the patient's chest in positions which form the second and third electrodes in the standard Eindhoven triangle formation. A wristband is provided for securing the apparatus to the patient's wrist and a microprocessor controls the electronic circuitry such that the patient's ECG signal is monitored/recorded from six standard input channels. A modem device is attachable to the apparatus for effecting burst mode transmission of data to an external receiver.

In the device proposed by Righter *et al*., reduced data is collected from only three electrodes attached to the patient's chest and a fourth electrode attached to his or her wrist data using six different channels (from the six standard ECG lead configurations). However, it is to be noted that these six channels are input channels which feed signals selectively to a microprocessor within the device for further processing. Thus, as explained at col. 5, lines 19ff, by changing the values of the three select signals, the ECG recorder can record ECG data from any one of the multiple data input channels. Thus, data can be recorded from only one input channel at a time. Furthermore, as stated at col. 6, lines 5ff, during normal operation of the ECG data monitor/recorder, the microprocessor receives ECG data and stores it in sequential memory address locations in a digital memory. The microprocessor selectively records any one of the six channels of input by setting the select signals M1, M2 and M3 at values that correspond to the desired lead configuration. In a one minute cycle, all six input channels will be recorded.

It is thus apparent that the device disclosed in US Patent 5,365,935 must still be held steady by a patient for a whole minute in order for all the ECG data to be recorded. It is also clear that data is collected from the six channels separately and that no attempt is made to transmit the data in reduced time so as to shorten the time required by the patient to hold the device steady.

US Patent No. 5,343,870 (Gallant *et al*.) discloses a Holter-type recorder unit for use with an ambulatory ECG recording system. The recorder features real-time beat differentiation, including ST analysis and paced beat analysis of two or three channel sampled electrocardiogram data. The recorder also features real-time coding of beat morphology and summary information on cassette tape. Further, summary information for an entire analysis can be compiled and reverse recorded on to a cassette tape at the end of the analysis to allow downloading thereof into an ECG scanner while the cassette is being rewound.

It is to be noted that such a device is intended for permanent attachment to a patient so as to monitor heart activity continuously and to record on a cassette tape abnormalities as and when they occur in real time, so as to be capable of subsequent analysis by a doctor. Thus, reducing the time during which the ECG monitor must be attached to the patient is not an issue so far as this patent is concerned. Likewise, whilst data is collected from a plurality of input channels, there is no need nor suggestion to provide multiple output channels, since the speed of data recordation is of subsidiary concern.

It has to be understood that within the context of portable ECG monitors there exist two different philosophies. According to one approach, measurements are taken quickly using multiple input channels and then stored in memory, prior to transmitting to the remote monitoring center. In such case, of course, the data transmission is not effected in real time and the patient does not need to hold the device steady during actual transmission of the data but only during measurement, which may be fast. As against this, the patient in effect, is given control as to when and even whether to transmit the recorded data to the remote monitoring center.

The present Applicant currently deals with patients on an ambulatory basis for patients who are mobile such that patients who feel unwell can initiate communication with the remote monitoring center. It is considered in such case better to leave any decision regarding the patient's health to the physician and to this end it is considered preferable to ensure that data recorded by a patient must be sent to the remote monitoring center. This is best achieved by measuring and transmitting the ECG data in real time and not buffering the data before transmission. Since the transmission of the data is relatively time-consuming compared with the measurement itself, the problem arises how to maintain the device steady during transmission and, following on from this, how to reduce the transmission time.

It thus emerges that the prior art relates to ambulatory ECG recording systems and devices having multiple input channels. However, less effort appears to have been directed to reducing the time taken for complete ECG data to be transmitted from a patient to a monitoring center in real time. There is therefore a need to provide an ECG monitor where full ECG data is transmitted in real time, whilst requiring the patient to maintain the device steady for significantly reduced time. Not only would this be beneficial to the patient, but it would also allow the monitoring center to receive a patient's ECG data in shorter time and thus become available to other patients more quickly.

U.S. Patent No. 4,889,134 (Greenwold *et al*.) describes a device for measuring electrical activity of the heart of a user. The device includes a digital memory for storing an ECG record prior to its being transmitted to a remote monitoring center. A parallel mode of operation is described in col. 4, lines 29*ff* wherein the outputs from three channels are simultaneously read from the digital memory of the device and processed so as to be output in parallel over the telephone lines. A sequential mode of operation requires that the three signals be sent one after the other and be reconstructed by a practitioner at the receiving end. However, such reconstruction requires cutting and pasting the received signals and is difficult to achieve, albeit not impossible, owing to the difficulty in aligning the three signals. Thus, the device disclosed by Greenwold *et al*. favors parallel transmission, in order to avoid the need for synchronization of the three signals at the receiving end. However, it is not directed to real-time measurement of an ECG signal, but rather to a device where pre-stored data is played back over the telephone line to the practitioner. Nor does there appear to be any suggestion to transmit the rhythm strip continually on a dedicated channel.

It is also known that certain of the subscribers to the medical monitoring unit are particularly vulnerable to attack or seizure such as, for example, the elderly and infirm. To this end, it is also known to provide such people with panic alarm devices so that at the onset of an attack, in whatever form that might be, or even in the event of imminent risk thereof, the panic alarm device may be manually operated so as call for help in time of need and inform the central monitoring unit. Upon receipt of a panic signal, the central monitoring unit may then take appropriate action. Panic alarm devices are, therefore, an important component in the arsenal of those who are vulnerable, thereby providing them with greater security and self-confidence.

Typically, such panic alarms include a manually operated RF transmitter energized by a miniature hearing-aid type battery and which, when operated, sends an encoded signal characteristic of the user. By such means, a remote monitoring unit, upon receiving the encoded signal, knows from where the signal emanated. Most simply, the coding can be by way of modulating an RF signal with data representative of the user's personal code so that the received signal is indicative of the sender.

Panic buttons of this kind and the ECG monitors discussed above are typically separate devices. Patients who are considered at risk of heart failure by definition belong to the patient population for whom panic buttons are advisable, thus requiring such patients to equip themselves with both devices and to carry both devices on their person at all times. This is inconvenient and raises the risk that, in a moment of haste or forgetfulness, the patient may forget to take at least one of the devices on his or her person.

U.S. Patent No. 4,958,645 (Cadell *et al*.) discloses a wireless multiparameter medical telemetry system with means for identifying the immediate location of a patient. Thus, at least one communication channel is used to transmit location data and remaining channels are used to transmit signals representative of measured physiological signals. These signals may include ECG but there is no mention of obtaining a 12 lead ECG nor is there any attempt to reduce transmission time of an ECG or any other physiological signal by using multiple channels.

U.S. Patent No. 5,919,141 (Money *et al*.) discloses a compact multiparameter monitor for hospital use that can monitor many parameters including two ECG leads which is typical for in-hospital monitoring. The information can be transmitted in real time. Two channels of ECG waveform monitoring are employed, each relating to a respective interface circuit 22 and 23 that is used to receive two bipolar leads each from first and second channel ECG transducers 102 and 103 [col. 5, lines 28-31]. Although wireless transmission is used, there is no attempt to reduce transmission time nor to send a 12 lead ECG.

EP 0657136 (Casio) discloses a portable electrocardiograph having a foldable housing having means for converting an ECG signal to an acoustical tone for transmission over a telephone. The foldable feature allows the electrodes to be conveniently applied during use. There is no suggestion to dimension the housing and its angular displacement so as to define medically acceptable electrode positions to obtain the monitoring locations for the V1 and V2 electrode locations in a 12-lead ECG. The ECG measurements are made between two electrodes on the housing so as to provide a rhythm strip rather than the 12 lead electrocardiogram.

WO 9719631 (Beitler) discloses a multi-electrode, weighted, flexible belt that depends upon gravity to press the electrodes in place. Switched electrode groups allow selection of differently dimensioned sets of electrodes to be selected for different patients.

WO 9403106 (Reinhold) discloses a portable 12-lead electrocardiogram having an electrode support 12. The left arm (LA) electrode is fixed an exterior surface 16 of the electrode support 12 but is not integral therewith and thus requires separate positioning during use.

### SUMMARY OF THE INVENTION

It is an object of the invention to provide a portable ECG monitor allowing standard twelve-lead ECG measurements to be transmitted to a monitoring center, whilst requiring a patient to maintain the device steady for significantly reduced time than provided for in hitherto-proposed devices.

This objective is realized in accordance with a broad aspect of the invention by means of a portable ECG signaling device, comprising:
a housing supporting a plurality of chest electrodes and for connecting to at least one limb electrode for affixing to different parts of a patient's body so as to measure the patient's rhythm strip and 12-lead ECG, and
an ECG signaling circuit within the housing for collecting and transmitting fractional ECG data on at least two output channels in parallel, thereby allowing the 12-lead ECG data to be transmitted in less time than could be done by collecting and transmitting the 12-lead ECG data serially on a single output channel in real time.

In operation of the device, the ECG signaling circuit transmits on a first output channel the rhythm strip measured with two of the electrodes, and transmits on at least second and third output channels respective samples or functions thereof measured by predetermined ones of the chest and limb electrodes.

In particular, the invention allows the patient's rhythm strip to be continually transmitted on one channel together with split transmission of other data on the remaining channels, in such manner that the monitoring unit receiving the data can reconfigure the patient's ECG.

According to a preferred embodiment, the housing is book-shaped and includes a spine and two sections capable of limited rotation about the spine between respective closed and open positions. The chest electrodes include a respective pair of displaced first and second electrodes on an edge of each of the sections opposite the spine, and at least one selector switch is accessible from an external surface of the housing and is connected to the ECG signaling circuit and to each pair of displaced first and second electrodes for connecting either the first electrode of each pair or the second electrode of each pair to the ECG signaling circuit according to whether the patient is male or female.

In order to allow the patient to relay the ECG signal to a remote monitoring unit, a vocalizing unit may be provided for converting the ECG signal to a representative acoustic signal that can be sent over the telephone to the monitoring unit. Alternatively, the ECG signal may be modulated on to an RF carrier signal for direct transmission with the monitoring unit, thus not requiring that the patient be in ready access with a telephone. Likewise, the ECG signal can be transduced for direct transmission using a cellular telephone integrated within the ECG monitor.

### BRIEF DESCRIPTION OF THE DRAWINGS

Preferred embodiments of the invention will now be described, by way of non-limiting example only, with reference to the accompanying drawings, in which:
**Fig. 1** shows pictorially an ECG signaling device according to the invention when in use;
**Figs. 2, 3 and 4** shows pictorially different views of the ECG signaling device;
**Fig. 5** is a block diagram showing functionally the principal components in the ECG signaling device;
**Fig. 6** is a block diagram showing functionally possible audio output channels in the ECG signaling device;
**Figs. 7a and 7b** are a flow diagram showing the principal operating steps carried out by a micro-controller in the ECG signaling device; and
**Fig. 8** is a pictorial representation of a system employing the ECG signaling device of the invention in conjunction with a monitoring center having a receiver adapted to receive data from the ECG signaling device.

### DETAILED DESCRIPTION OF THE INVENTION

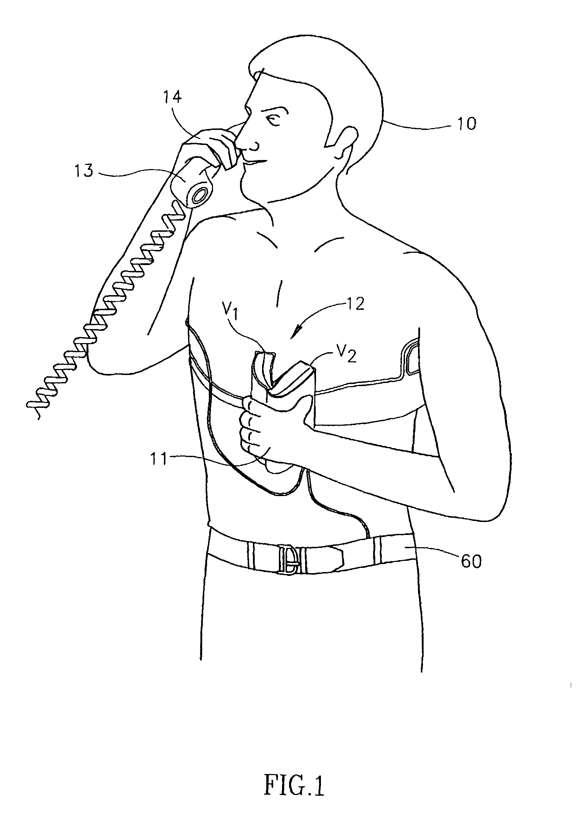

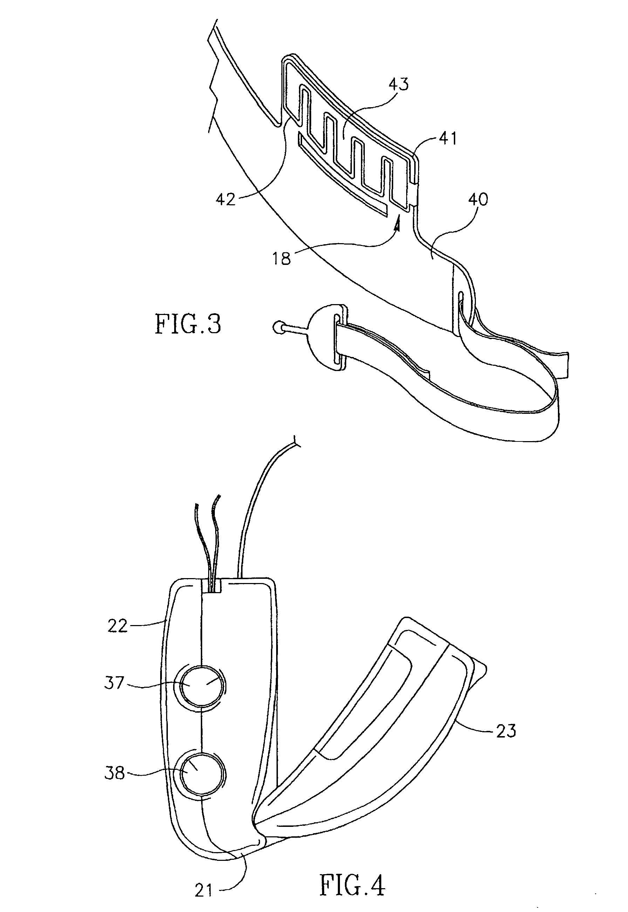

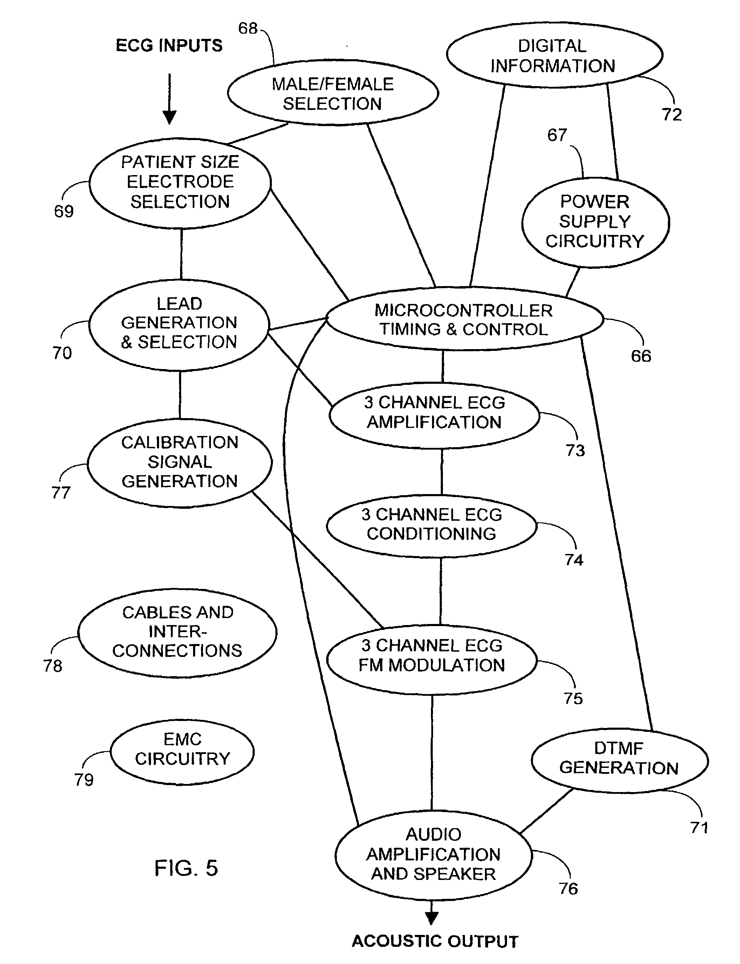

Figs. 1 to 4 show pictorially a patient 10 holding in his or her left hand 11 a portable ECG signaling device 12 according to the invention for transmitting acoustically ECG data via a telephone 13 held in the patient's right hand 14. The ECG signaling device 12 comprises a housing 15 supporting a plurality of chest electrodes 16 and limb electrodes 17, 18 and 19 for affixing to different parts of the patient's body. An ECG signaling circuit within the housing 15 measures the patient's rhythm strip and 12-lead ECG, and transmits fractional ECG data on at least two output channels in parallel. This allows complete ECG data to be transmitted in less time than could be done by transmitting the complete ECG data serially on a single channel. The ECG signaling circuit is described is further detail below with reference to Figs. 5 to 7 of the drawings.

The housing 15 is book-shaped and includes a spine 21 and two sections 22 and 23 capable of limited rotation about the spine between a respective closed position (not shown) and open position as shown in Figs.1, 2 and 4. The chest electrodes include a respective pair of displaced first electrodes 24, 24' and second electrodes 25, 25' respectively, on a respective edge 26, 26' of each of the sections 22 and 23 opposite the spine 21. A pair of displaced color-coded selector switches 30 and 31 (constituting at least one selector switch) is accessible from an external surface of the housing 15 for operation by male and female patients, respectively. The selector switches are constructed so as be easily operable from any position of the switch, so as to be suitable for patients having hands of different sizes. The selector switches 30 and 31 are connected to the ECG signaling circuit within the housing and to the first electrodes 24, 24' and second electrodes 25, 25' for connecting one of the first electrodes 24, 24' and a corresponding one of the second electrodes 25, 25' to the ECG signaling circuit according to whether the patient 10 is male or female.

The limb electrodes include first and second underarm electrodes 17 and 18 and a leg electrode 19 connected to the ECG signaling circuit. The first underarm electrode 17 is connected by a thin cable 32 to the ECG signaling circuit and is placed by the patient 10 under his or her right armpit, Likewise, the leg electrode 19 is connected by a thin cable 33 to the ECG signaling circuit and is placed on the patient's waist where it may be secured by a belt worn by the patient. If desired, the ECG signaling circuit may include connector sockets (not shown) accessible from outside the housing 15 for removably connecting the first underarm electrode 17 and the leg electrode 19 thereto. Alternatively, the first underarm electrode 17 and the leg electrode 19 may be connected permanently to the ECG signaling circuit.

The chest electrodes 16 are supported in spaced relationship on a first surface 35 of a thin, flexible electrode support 36. The chest electrodes 16 may be formed by a screen-printing technique, although any other suitable method for fixing the electrodes to a flexible, insulating liner may be employed. Some of the chest electrodes are redundant and the ECG signaling circuit includes a pair of rotary switches 37 and 38 one for male and one for female. The rotary switches 37 and 38 constitute size selector switches having a plurality of settings, each in respect of a different range of size of patient. The ECG signaling circuit is responsive to a selected setting of the size selector switch for selecting different ones of the chest electrodes. Some of the chest electrodes may be assigned for male use exclusively and others are for female use exclusively. The flexible electrode support 36 is foldable into a compact assembly when not in use.

The first underarm electrode 18 is disposed on a second surface 40 of the electrode support 36 opposite the first surface thereof and projects therefrom as an elongated tongue portion 41 extending along a length of the electrode support. The first underarm electrode 18 includes a plurality of spatially separated tines 42 connected to a common support electrode 43 that extends along a full width of the tongue portion 41. Such a construction ensures that in use at least one of the tines 42 will be securely situated under the patient's left armpit regardless of the patient's size. The spacing between adjacent tines 42 is dimensioned to allow the tines to be folded around the housing when not in use.

A slot 44 at an end of the support 36 remote from the housing 15 accommodates therein an adjustable elastic belt 45 (constituting a fastener) for securing the device around the patient's body. To this end, the belt has a clasp 46 at its fee end for engaging a keyhole 47 formed in the housing 15 and thereby activating a microswitch (not shown). The ECG signaling circuit is responsive to a status of the microswitch for determining whether the belt 45 is fastened or not, and producing a corresponding indication signal. In use, the housing may be fully opened to a predetermined maximum of approximately 36°, and in order to prevent operation of the device until it is fully opened, a reed switch is mounted in one of the sections 22 and a magnet is mounted in the other section 23. When the device is closed, the reed switch is closed and prevents operation of the ECG signaling circuit. The ECG signaling circuit is responsively coupled to the first and second underarm electrodes 17 and 18 and to the leg electrode 19 in conjunction with selected ones of the chest electrodes 16, 24 and 25 for producing a full twelve-lead electrocardiogram.

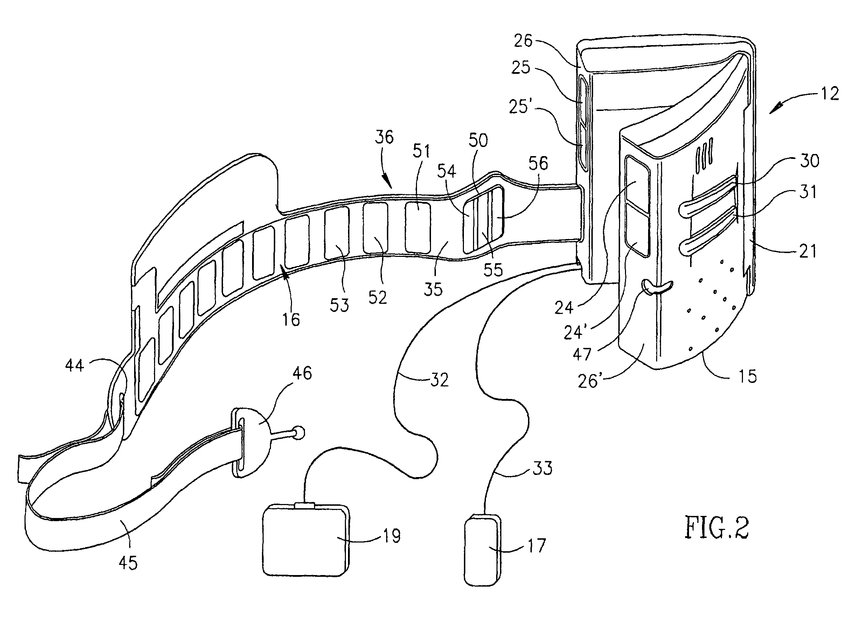

Referring to the universally adopted symbols V₁, V₂, V₃, V₄, V₅ and V₆, LA, RA and LL as employed in US Patent No. 5,339,823 (Reinhold, Jr.), the electrode pairs 24, 24' and 25, 25' correspond to V₁ and V₂, respectively. The distance between these electrodes is dictated by the relevant standard and is automatically achieved when the sections 26 and 26' are fully opened. To this end, the two sections 26 and 26' may be resiliently biased so that they cannot be opened only partially. As shown in Fig. 2, the chest electrodes 16 also include electrodes 50 and 51, which correspond to V₃ and V₄, respectively. The V₃ electrode 50 should be in mid-geometrical position (measured horizontally) between V₂ and V₄, i.e. between the electrodes 25 or 25' and 51. In practice, however, electrode 52 and 53 may also serve as V₄, switching between the electrodes 51, 52 and 53 being effected by the size selector switches 37 and 38. In order to ensure that the V₃ electrode 50 be in mid-geometrical position between V₂ and V₄, it is necessary to move the effective position of the V₃ electrode 50 depending on which of the V₄ electrodes 51, 52 or 53 is operative. To this end, the V₃ electrode 50 is divided into three mutually insulated sections 54, 55 and 56, only one of which is selected depending on the setting of the size selector switches 36 and 37, whereby the selected V₃ electrode section is mid-way between the V₂ electrode and the operative V₄ electrode.

Fig. 1 shows how the device 12 is used for the determination of a full twelve-electrode ECG measurement. The patient 10 opens the housing and holds the edges 26 and 26' against his or her chest so that the leads V₁ and V₂ are substantially symmetrically disposed about his or her vertebrae. The electrode support is dimensioned for placement against a patient's bare chest and different electrodes in the electrode support 16 are selected according to the selected patients size. Having affixed the two electrodes 24 and 25 (or 24' and 25') corresponding to V₁ and V₂, the four electrodes V₃, V₄, V₅ and V₆ are now disposed on the patient's left rib cage, being mutually displaced by the required distance appropriate to the patient so that the six electrodes V₁ to V₆ serve as Wilson precordial electrodes. The dimensions of the electrode support 36 are such that for a given patient, the arm electrode 17 fits under the patient's right armpit, whilst at least one of the sections of the electrode 18 is held under the patient's left armpit. The electrode 19 is then fitted near the patient's waist, typically being held in place by a belt 60 as shown in Fig. 1.

Fig. 5 shows functionally an ECG signaling device 65 according to the invention, comprising a micro-controller 66 energized by a power supply 67 and responsively coupled to a Male/Female selection switch 68 and patient size electrode selection switch 69. The latter is connected to the arm, leg and chest electrodes as described above with reference to Figs. 1 to 4 of the drawings. The micro-controller 66 is connected to a lead generation and selection unit 70, to a DTMF generation unit 71 and to a digital information unit 72. Also coupled to the micro-controller 66 is a 3-channel ECG amplifier 73 coupled to a 3-channel ECG conditioning unit 74, a 3-channel ECG FM Modulator 75 and an audio amplifier and speaker 76. Connected between the lead generation and selection unit 70 and the 3-channel ECG FM Modulator 75 is a calibration signal generator 77. Also provided are cables and interconnections 78 and EMC circuitry 79. These components will now be described in greater detail.

### Power Supply Circuitry

The power supply circuitry uses a 9 Volt battery to provide a regulated DC voltage (V_{cc}) to allow effective and efficient amplification and processing of the ECG signals. The DC voltage level (V_{cc}) is regulated at 6.5 Volts DC.

As the ECG signal to be amplified is a bi-polar signal, an analog ground is required. The analog ground level is approximately midway between the regulated voltage supply rails. As the regulated voltage level (V_{cc}) is 6.5 Volts and the battery ground level is 0 Volts, the analog ground level (AGND) is fixed at 2.8 Volts allowing a substantial voltage swing either side of this analog ground.

The power supply circuitry detects the low voltage level prior to transmission, thus minimizing the chance of the regulator switching off the output during a transmission. A low battery voltage cut-off level set in the region of 7.5 Volts DC, ensures that the ECG signaling circuit 65 cannot operate if the battery voltage falls below this threshold.

### Patient Size Electrode Selection

The circuitry is to measure the 12-Lead ECG from the V leads which are selected as a combination of V₁ and V₂ electrodes mounted on the housing 15 and the electrodes mounted on the electrode support 36. As noted above, the combination of electrodes used depends on the size and gender of the patient. The patient size may be set to one of four pre-set sizes using the size selector switches 37 and 38, one for male and the other for female. Each size uses a pre-defined combination of electrodes. Depending on which operation button 30 or 31 is depressed (male/female), patient size is defined by the respective size switch 37 or 38.

The micro-controller 66 reads the size switch prior to transmission and selects the correct combination of electrodes from the electrode support using a combination of multiplexers. The selected electrodes remain active during the period of the transmission.

### Male/Female Selection

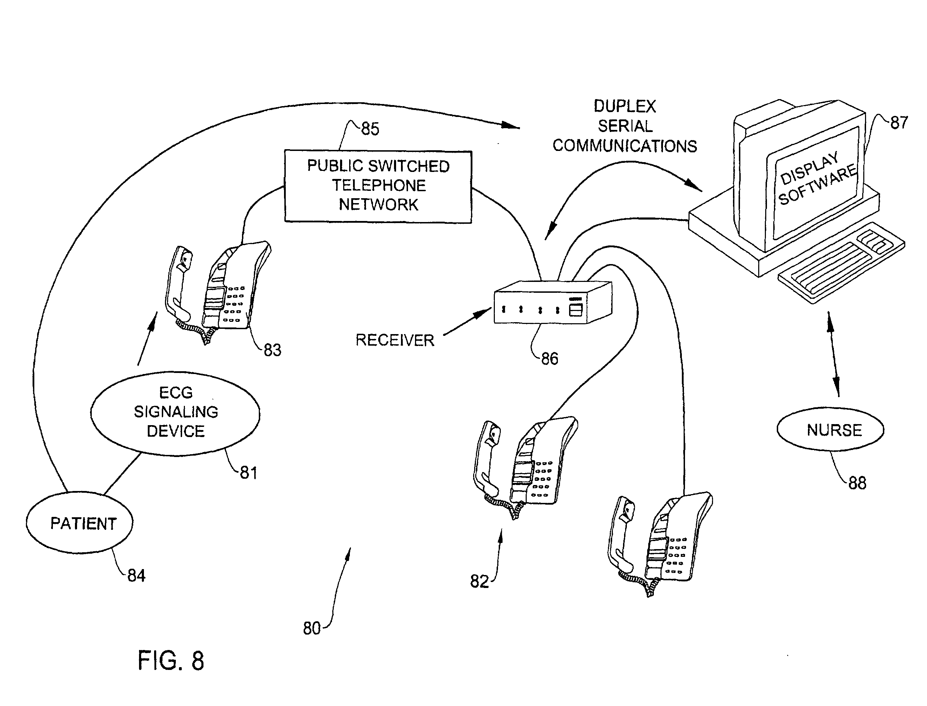

There exist two modes of transmission, male or female, which are initiated by pressing either the male or female operating button 30 or 31. The operating button activates a switch that is held on for a short period to allow latching on of the circuit. After the circuit has been latched on, the operating button 30 or 31 may be released and the circuit completes the initiated transmission. During the 'latching on' period, the transmitter transmits a 2,850 Hz tone so that the receiver at the monitoring center may detect that the incoming signal is from an ECG signaling device according to the invention. Other types of device are identified by a different frequency tone. The monitoring center is described below with reference to Fig. 8 of the drawings.

If the male operating button 30 is depressed, the micro-controller determines which electrodes are to be used for ECG measurement for the selected size of male patient as set by the size selector switch 37. Conversely, if the female operating button 31 is selected, the micro-controller determines the correct combination of electrodes to be monitored for the selected size of female patient as set by the size selector switch 38. As part of the digital information to be transmitted prior to the ECG signal, the micro-controller 66 monitors which operating button 30 or 31 has been depressed and transmits this information in digital format with the digital information.

### Lead Generation and Selection

The combination of electrodes used to select the required ECG signals is selected by multiplexers under the control of the micro-controller 66. The 12-Lead ECG and rhythm strip signals are selected using the electrodes as tabulated below.

| **Lead** | | **Electrode Combination** |
|---|---|---|
| Lead I | Bipolar Limb Leads (Einthoven) | LA - RA (*left arm minus right arm*) |
| Lead II | | LL - RA (*left leg minus right arm*) |
| Lead III | | LL - LA (*left leg minus left arm*) |
| V1 | Unipolar Chest Leads (Wilson) | V at Size Selected V1 Electrode, V1=V-0.333 (LA+RA+LL) |
| V2 | | V at Size Selected V2 Electrode, V2=V-0.333 (LA+RA+LL) |
| V3 | | V at Size Selected V3 Electrode, V3=V-0.333 (LA+RA+LL) |
| V4 | | V at Size Selected V4 Electrode, V4=V-0.333 (LA+RA+LL) |
| V5 | | V at Size Selected V5 Electrode, V5=V-0.333 (LA+RA+LL) |
| V6 | | V at Size Selected V6 Electrode, V6=V-0.333 (LA+RA+LL) |

The timing of the transmission of each lead is controlled by the micro-controller 66. Before each lead is transmitted, an identifying signal is transmitted in digital format. A digital '1' is sent as a 100ms signal equivalent to +1mV dc input. Conversely, a digital '0' is transmitted as a 100ms signal equivalent to -1mV dc input. The equivalent +1mV and -1mV signals are switched by the micro-controller.

### Calibration Signal Generation

Two calibration signals are generated which are the equivalent of a +1mV and a -1mV signal measured on the chest. The calibration signal is generated with reference to the analog ground level. The +1mV calibration signal is stable within ± 2.5%, and should be buffered while the -1mV signal may be generated by using a unity gain inverting buffer on the same signal.

The calibration signals are applied directly to the frequency modulation circuitry 75. The magnitude of the calibration signal is dependent on the gain of the instrumentation amplifier stage (e.g. if the instrumentation amplifier has a gain of 500, the input to the FM Modulation stage should be 0.5Volt).

### Digital Information

Digital information as shown in the following table is sent prior to the ECG transmission in Dual Tone Multiple Frequency (DTMF) format. The digital information includes both programmed data (serial number and model number) programmed during manufacture, and hardware configuration data. The hardware configurations can be changed between transmissions, and therefore the hardware configuration is assessed prior to transmission and the details are sent as digital data. The DTMF circuitry is directly controlled by the micro-controller 66 and is switched to the audio amplifier stage during DTMF transmission. The ECG transmission circuitry (3-channel FM signal) is switched away from the audio amplifier stage during DTMF transmission.

| **Hardware** | **Description** |
|---|---|
| Battery Condition | The micro-controller monitors the battery voltage prior to transmission and sends a DTMF digit indicating battery level. |
| Device ID and Model No. | Allows the monitoring center to identify the patient and verifies that the correct selector switch was pressed. |
| Operation Button (male/female) | If the male operation button is depressed, a 'high' signal is to be sent to the micro-controller and *vice versa*. |
| Configuration of size selector switch (four settings) | Depending on which operation button was depressed (male or female), the micro-controller receives a digital code from the respective size selector switch (male or female) indicating which size has been selected. A DTMF digit is transmitted indicating size. |
| Electrode Belt Attached (Normal or Extra Small) | Wiring is provided on the electrode belt so that the micro-controller may monitor a point to tell whether the belt fitted is normal or extra small. If the normal belt is fitted, the monitoring point is 'low'. If the extra small belt is fitted, wiring on the belt drives the monitoring point 'high'. |
| RF Transmitter (fitted or not) | |
| Elastic Belt Status | A switch is mounted within the key recess 47 of the housing used to attach the elastic belt thereto. When the elastic belt is fixed around the body and the clip is attached, the micro-controller monitoring point is driven 'high'. If the elastic belt is not fixed around the body, the monitoring point remains 'low'. |

### Three Channel ECG Amplification

Each of the three channels has its own ECG amplifier circuit based on a precision differential amplifier design and having the capability of amplifying ECG (mV) signals. Each ECG amplifier stage has a gain of 10. The common mode rejection ratio (CMRR) is at least 80dB and the system noise is less that 40µV r.t.i.

The dynamic range of each ECG determines the amplitude of ECG signal that may be frequency modulated and transmitted. The dynamic range is approximately ± 2.5mV, but a more optimal use of the available channel bandwidth may be used to achieve a better deviation sensitivity. Beyond the dynamic range, the amplified signal is clipped and held at the dynamic range level.

### Three Channel ECG Conditioning

Each of the three channels is conditioned to provide a high quality ECG signal. The frequency content of the ECG signal should be 0.05 - 150Hz and this is ensured by both high pass (0.05Hz) and low pass (150Hz) filtering. Circuitry is included for pace pulse stretching which converts a very short duration pace pulse to a wider pulse suitable for transmission by the ECG signaling device and for detection by a receiver in the remote monitoring center.

Baseline stabilization is included so that the offsets due to muscle artifact, breathing and electrode EMF are minimized. Baseline stability is particularly important in the ECG signaling device because of the restrictions on dynamic range.

### Three Channel ECG FM Modulation

Each channel frequency modulates the amplified ECG signal, using a Voltage Controlled Oscillator configuration. Each generated Frequency Modulated carrier approximates a sinusoidal waveform thus minimizing the harmonic content.

In order to make optimum use of the available frequency spectrum, each channel is frequency modulated around a center frequency set to 1275, 1875 and 2850Hz, respectively. As well as the ECG signals, the FM modulation circuitry processes the generated calibration signals. Each Voltage Controlled Oscillator (VCO) has a gain set to define the relationship between the input (mV) signal and the change in frequency at the FM output. This gain (called the "deviation sensitivity") is set to be 60Hz/mV. These features are summarized in the following table.

| **Channel** | **Center Frequency** | **Dynamic Range** | **Deviation Sensitivity** |
|---|---|---|---|
| Channel 1 | 1275 Hz ± 10 Hz | ±2.5mV | ±60Hz/mV |
| Channel 2 | 1875 Hz ± 10 Hz | ±2.5mV | ±60Hz/mV |
| Channel 3 | 2850 Hz ± 10 Hz | ±2.5mV | ±60Hz/mV |

### Audio Amplification and Speaker

The frequency modulated signals from each channel are mixed together and amplified to produce an acoustic signal. The mixing and amplification is performed in the audio amplifier stage. During mixing, each channel is weighted to assist in the transmission power levels of each channel. The audio amplifier is designed to drive the chosen speaker to produce adequate acoustic power while minimizing current consumption to prolong battery life span. The audio amplifier is designed so that the signals are weighted to ensure effective acoustic transmission from the transmitter speaker to the telephone, and from the telephone through the telephone line to the receiver.

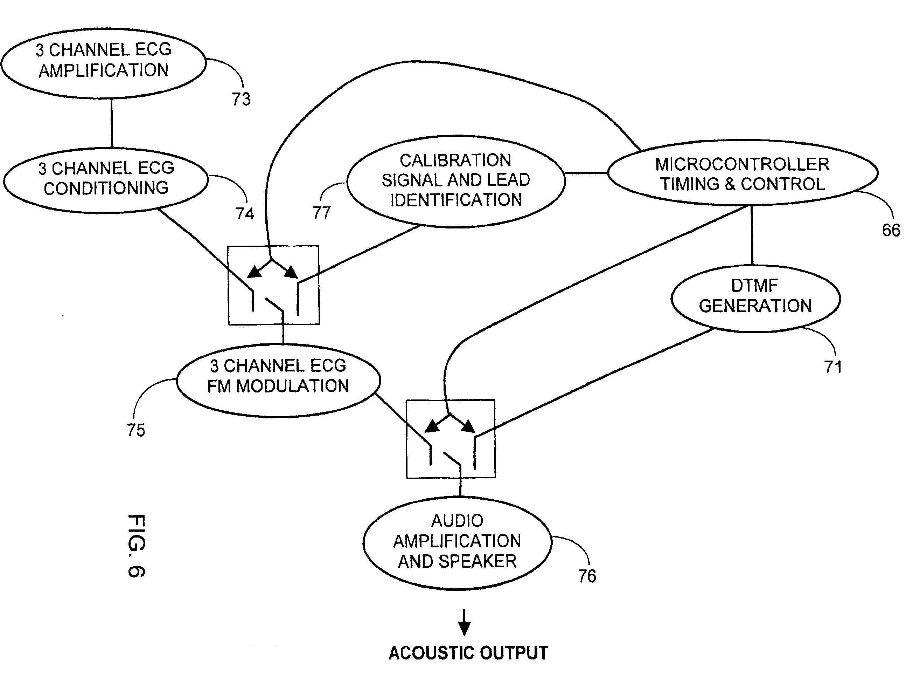

Fig. 6 summarizes the switching of the audio amplification and speaker 76, which may be fed one of three different acoustic signals. A signal may be fed from the three channel ECG conditioning unit 74, after FM modulation, so as to derive the ECG signal on the selected channel. Likewise, the calibration signal and lead identification signal may be fed thereto, also after FM modulation; or a DTMF signal may be fed to the audio amplification and speaker 76 via the DTMF generation unit 71.

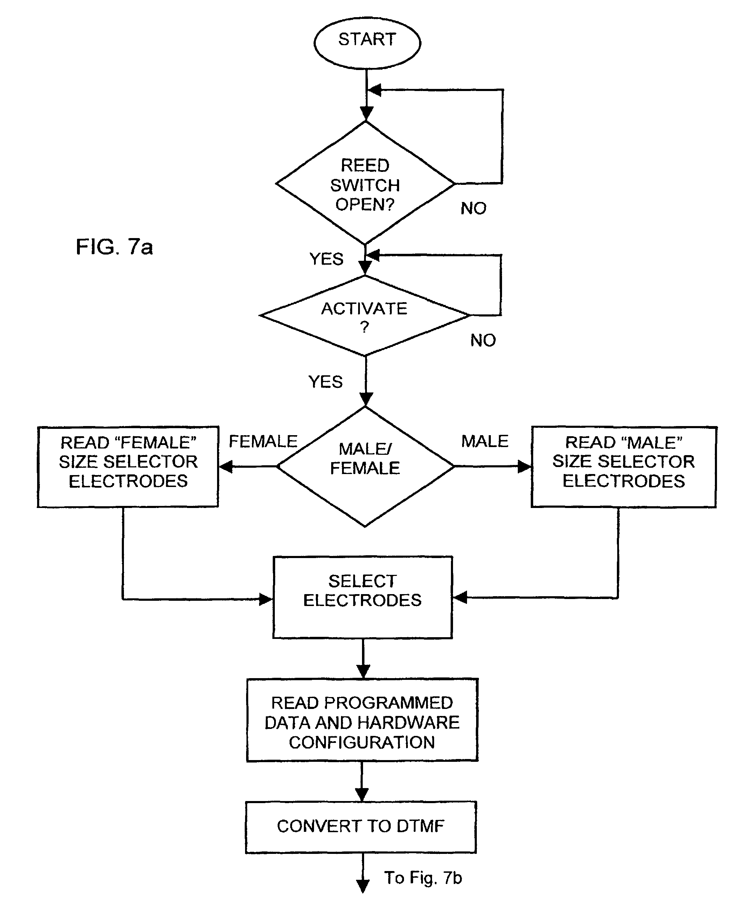

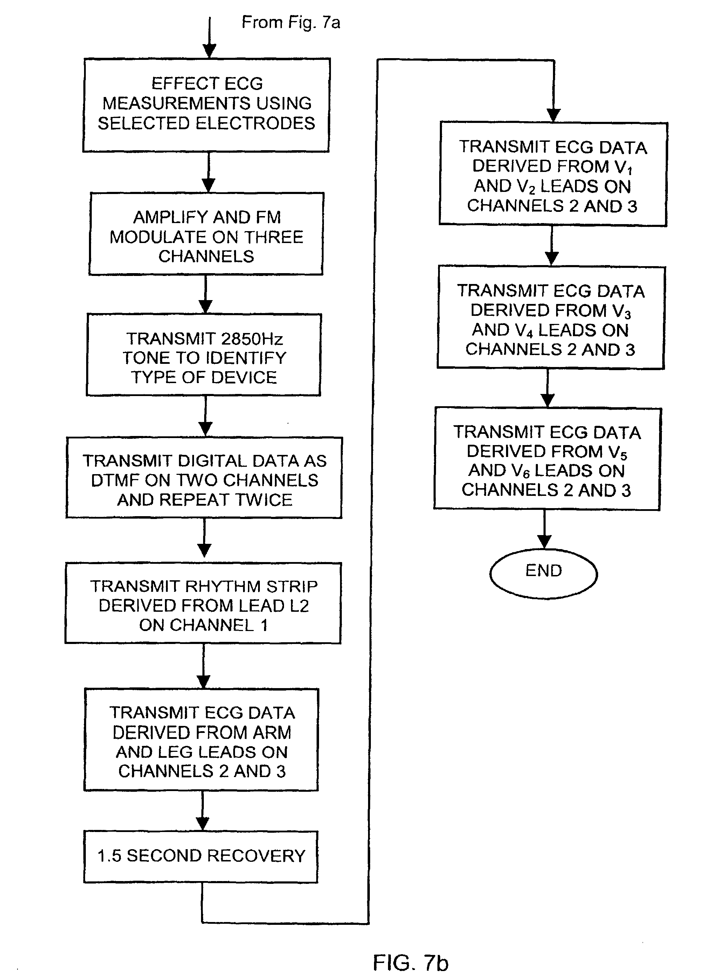

Figs. 7a and 7b summarize the principal steps taken by the micro-controller 66 shown in Fig. 5. Thus, initially the micro-controller checks that the housing is open and activated. Thereafter, the micro-controller checks whether the male or female selector switch has been depressed and reads the appropriate size selector switch settings, so as to select the appropriate electrodes. The micro-controller then reads the programmed data and hardware configuration and converts to a DTMF signal. The ECG measurements are effected using the selected electrodes, amplified using three separate amplifiers and frequency-modulated using three separate FM modulators. A tone of 2,850Hz is transmitted so as to identify the type of ECG signaling device being used by the patient and the digital data is then transmitted as DTMF signals on two separate audio channels. This is repeated twice so that, in the event of one or even two of the data transmissions being garbled, the monitoring center still receives valid data. Thereafter, the rhythm strip derived from the lead L2 is transmitted continuously on a first one of the channels. ECG data derived from the arm and leg leads are transmitted on leads L1 and L3. There then follows a 1.5 second recovery period during which no data are transmitted. Thereafter, on the second and third channels, ECG data derived from the V₁ and V₂ electrodes, the V₃ and V₄ electrodes and the V₅ and V₆ electrodes are transmitted successively during 2.5 second cycles.

Fig. 8 shows pictorially a detail of a system 80 using an ECG signaling device 81 in conjunction with a remote monitoring center 82 adapted for receiving data from the ECG signaling device 81. Data is transmitted acoustically by the ECG signaling device 81 through a telephone 83 operated by a patient 84 using the ECG signaling device 81 through the Public Switched Telephone Network 85 to a receiver 86 at the monitoring center 82. The receiver 86 reads the received data and reconstructs the patient's ECG and rhythm strip for visual display on a display monitor 87 for review by a nurse or other medical orderly 88.

As explained in detail above, with particular reference to Figs. 7a and 7b, the ECG signaling device 81 sends digital information in DTMF mode and sends ECG information using three channel FM modulation mode. The receiver 86 is programmed to read the DTMF and FM signals and reconstruct the data transmitted by the ECG signaling device, thereby deriving the patient's rhythm strip and ECG data. At the same time, the receiver 86 performs rudimentary verification of the received data. For example, since the patient 84 using the ECG signaling device 81 is registered with the monitoring center 82, the patient's sex is known to the monitoring center 82. The monitoring center 82 verifies that the selector switch selected by the patient is appropriate to the patient's sex and ignores any data if this is not the case (apart from taking manual remedial action to alert the patient). Likewise, although the size selector switches are not intended for adjustment by the patient, data relating to their settings are also transmitted and may be verified by the monitoring center as being appropriate for the registered patient.

It will also be understood that instead of transmitting the analog signals vocally, the signals may be converted by an A/D Converter to equivalent digital signals and then transmitted using standard digital techniques.

Further, whilst the invention has been described with particular regard to a portable ECG monitor, it is also envisaged that a portable ECG monitor can incorporate features of a panic alarm system, thereby obviating patients to carry separate devices on their person. Such a portable ECG monitor may conform to the device according to the present invention but clearly the novel concept of combining a portable ECG monitor with a panic alarm system is not restricted to any specific form of portable ECG monitor or panic alarm system.

It will be appreciated that modifications and variations may be effected to the preferred embodiment without departing from the invention.

## Claims

1. A portable ECG signaling device (12), comprising.
a housing (15) supporting a plurality of chest electrodes (16, 18) and for connecting to at least one limb electrode (17, 19) for affixing to different parts of a patient's body (10) so as to measure the patient's rhythm strip and 12-lead ECG, and
an ECG signaling circuit (65) within the housing for collecting and transmitting fractional ECG data on at least two output channels in parallel, thereby allowing the 12-lead ECG data to be transmitted in less time than could be done by collecting and transmitting the 12-lead ECG data serially on a single output channel in real time;
**characterized in that**:
in operation of the device, the ECG signaling circuit transmits on a first output channel the rhythm strip measured with two of the electrodes, and transmits on at least second and third output channels respective samples or functions thereof measured by predetermined ones of the chest and limb electrodes.

2. The portable ECG signaling device according to Claim 1, wherein each channel frequency modulates an ECG signal fed thereto around a respective center frequency chosen to make optimum use of an available frequency spectrum.

3. The portable ECG signaling device according to Claim 2, wherein the respective center frequencies are approximately 1275Hz, 1875Hz and 2850Hz.

4. The portable ECG signaling device according to any one of Claims 1 to 3, wherein a complete transmission sequence takes approximately 16 seconds.

5. The portable ECG signaling device according to any one of the preceding Claims, wherein the at least one limb electrode includes first and second underarm electrodes and a leg electrode capable of connection to the ECG signaling circuit.

6. The portable ECG signaling device according to Claim 5, wherein the chest electrodes (16) are supported on a thin, flexible electrode support (36) in spaced relationship and operate in conjunction with the first and second underarm electrodes and the leg electrode for producing a twelve-lead electrocardiogram.

7. The portable ECG signaling device according to Claim 6, wherein the flexible electrode support is foldable into a compact assembly when not in use.

8. The portable ECG signaling device according to any one of Claims 1 to 7, further including:
at least one selector switch (30, 31) accessible from an external surface of the housing and connected to the ECG signaling circuit and to respective pairs of displaced first and second electrodes for connecting one of the first electrodes and a corresponding one of the second electrodes of each pair to the ECG signaling circuit according to whether the patient is male or female.

9. The portable ECG signaling device according to Claim 8, wherein the at least one selector switch is constituted by displaced first and second selector switches (30, 31) for operation by male and female patients, respectively.

10. The portable ECG signaling device according to Claim 9, wherein the first and second selector switches are color-coded.

11. The portable ECG signaling device according to Claim 5, wherein:
the chest electrodes (16) are disposed on a first surface (35) of the electrode support, and
the first underarm electrode (18) is disposed on a second surface (40) of the electrode support opposite the first surface thereof.

12. The portable ECG signaling device according to Claim 11, wherein the first underarm electrode (18) extends along a length of the electrode support so as to adapt to patients of different sizes.

13. The portable ECG signaling device according to Claim 12, wherein the first underarm electrode (18) includes a plurality of spatially separated tines (42) connected to a common support electrode (43).

14. The portable ECG signaling device according to Claim 13, wherein a spacing between adjacent tines is dimensioned to allow the tines to be folded around the housing when not in use.

15. The portable ECG signaling device according to any one of Claims 5 and 11 to 14, wherein the first underarm electrode is adapted for location under a patient's left armpit.

16. The portable ECG signaling device according to any one of Claims 1 to 15,
wherein the electrodes are formed by a screen-printing technique.

17. The portable ECG signaling device according to any one of Claims 1 to 16,
wherein:
the plurality of chest electrodes (16) includes redundant electrodes,
the ECG signaling circuit includes a size selector switch (37, 38) having a plurality of settings each in respect of a different range of size of patient, and
the ECG signaling circuit is responsive to a selected setting of the size selector switch for selecting different ones of the chest electrodes.

18. The portable ECG signaling device according to any one of the preceding Claims, including a transmitter (76) for transmitting a signal representative of a patient's ECG.

19. The portable ECG signaling device according to Claim 18, wherein the transmitter includes a vocalizing unit for producing an acoustic signal representative of a patient's ECG.

20. The portable ECG signaling device according Claim 18, wherein the transmitter includes digital circuitry (71) for producing a digital signal representative of a patient's ECG

21. The portable ECG signaling device according Claim 19, further including a RF transmitter for transmitting an RF modulated carrier signal representative of a patient's ECG.

22. The portable ECG signaling device according to any one of the preceding Claims, further including a fastener (46) for securing the device around a patient's body.

23. The portable ECG signaling device according to any one of Claims 18 to 21, wherein the transmitter is further adapted to transmit data indicating at least some of the following parameters:
**(a)** a Model number of the portable ECG signaling device,
**(b)** an ID number of the portable ECG signaling device,
**(c)** condition of a battery within the portable ECG signaling device,
**(d)** an indication of which of the first and second electrodes was selected,
**(e)** selected size range of patient,
**(f)** fastener status,
**(g)** size of electrode support, and
**(h)** RF transmitter status.

24. The portable ECG signaling device according to any one of Claims 18 to 21, wherein the transmitter is adapted to transmit:
**(a)** a tone identifying the ECG signaling device,
**(b)** digital data identifying a hardware configuration of the ECG signaling device and pre-programmed data,
**(c)** the patient's rhythm strip being transmitted continuously on a first output channel,
**(d)** ECG data derived from the arm and leg electrodes being transmitted on second and third channels simultaneously,
**(e)** successive pairs of ECG data derived from the chest electrodes being transmitted on the second and third channels simultaneously.

25. The portable ECG signaling device according to any one of Claims 1 to 24, being integral with a panic alarm device.

## Patentansprüche

1. Tragbare EKG-Signalübertragungsvorrichtung (12), die umfasst:
ein Gehäuse (15), das eine Vielzahl von Brustkorbelektroden (16, 18) aufnimmt und mit wenigstens einer Gliedmaßenelektrode (17, 19) zur Befestigung an verschiedenen Teilen des Körpers eines Patienten (10) verbunden ist, um den Rhythmusstreifen des Patienten und ein 12-Ableitungs-EKG zu messen, und
eine EKG-Signalübertragungsschaltung (65) in dem Gehäuse zum Erfassen und Senden von Teil-EKG-Daten parallel auf wenigstens zwei Ausgangskanälen, um so das Senden des 12-Ableitungs-EKG in kürzerer Zeit zu ermöglichen, als dies durch serielles Erfassen und Senden des 12-Ableitungs-EKG auf einem einzelnen Ausgangskanal in Echtzeit möglich wäre;
**dadurch gekennzeichnet, dass**:
im Betrieb der Vorrichtung die EKG-Signalübertragungsschaltung auf einem ersten Ausgangskanal den mit zwei der Elektroden gemessenen Rhythmusstreifen sendet und auf wenigstens einem zweiten und einem dritten Ausgangskanal entsprechende Abtastwerte oder Funktionen desselben sendet, die durch vorgegebene der Brustkorb- und Gliedmaßenelektroden gemessen werden.

2. Tragbare EKG-Signalübertragungsvorrichtung nach Anspruch 1, wobei jeder Kanal Frequenzmodulation eines ihm zugeführten EKG-Signals um eine entsprechende Mittenfrequenz herum durchführt, die so gewählt wird, dass ein verfügbares Frequenzspektrum optimal genutzt wird.

3. Tragbare EKG-Signalübertragungsvorrichtung nach Anspruch 2, wobei die entsprechenden Mittenfrequenzen ungefähr 1275 Hz, 1875 Hz und 2850 Hz betragen.

4. Tragbare EKG-Signalübertragungsvorrichtung nach einem der Ansprüche 1 bis 3, wobei eine komplette Sende-Sequenz ungefähr 16 Sekunden dauert.

5. Tragbare EKG-Signalübertragungsvorrichtung nach einem der vorangehenden Ansprüche, wobei die wenigstens eine Gliedmaßenelektrode eine erste und eine zweite Unterarmelektrode sowie eine Beinelektrode enthält, die mit der EKG-Signalübertragungsschaltung verbunden werden können.

6. Tragbare EKG-Signalübertragungsvorrichtung nach Anspruch 5, wobei die Brustkorbelektroden (16) von einem dünnen flexiblen Elektrodenträger (36) in beabstandeter Beziehung getragen werden und zusammen mit der ersten sowie der zweiten Unterarmelektrode und der Beinelektrode arbeiten, um ein 12-Ableitungs-Elektrokardiogramm zu erzeugen.

7. Tragbare EKG-Signalübertragungsvorrichtung nach Anspruch 6, wobei der flexible Elektrodenträger, wenn er nicht in Gebrauch ist, zu einer kompakten Anordnung zusammengeklappt werden kann.

8. Tragbare EKG-Signalübertragungsvorrichtung nach einem der Ansprüche 1 bis 7, die des Weiteren enthält:
wenigstens einen Wählschalter (30, 31), der von einer Außenfläche des Gehäuses zugänglich und mit der EKG-Signalübertragungsschaltung sowie entsprechenden Paaren versetzter erster und zweiter Elektroden verbunden ist, um je nachdem, ob der Patient ein Mann oder eine Frau ist, eine der ersten Elektroden und eine entsprechende der zweiten Elektroden jedes Paars mit der EKG-Signalübertragungsschaltung zu verbinden.

9. Tragbare EKG-Signalübertragungsvorrichtung nach Anspruch 8, wobei der wenigstens eine Wählschalter durch einen versetzten ersten und zweiten Wählschalter (30, 31) zur Bedienung durch männliche bzw. weibliche Patienten gebildet wird.

10. Tragbare EKG-Signalübertragungsvorrichtung nach Anspruch 9, wobei der erste und der zweite Wählschalter farbcodiert sind.

11. Tragbare EKG-Signalübertragungsvorrichtung nach Anspruch 5, wobei:
die Brustkorbelektroden (16) an einer ersten Fläche (35) des Elektrodenträgers angeordnet sind, und
die erste Unterarmelektrode (18) an einer zweiten Fläche (40) des Elektrodenträgers gegenüber der ersten Fläche desselben angeordnet ist.

12. Tragbare EKG-Signalübertragungsvorrichtung nach Anspruch 11, wobei die erste Unterarmelektrode (18) sich über eine Länge des Elektrodenträgers erstreckt, um sie an Patienten mit unterschiedlicher Größe anzupassen.

13. Tragbare EKG-Signalübertragungsvorrichtung nach Anspruch 12, wobei die erste Unterarmelektrode (18) eine Vielzahl räumlich getrennter Zacken (42) enthält, die mit einer gemeinsamen Trägerelektrode (43) verbunden sind.

14. Tragbare EKG-Signalübertragungsvorrichtung nach Anspruch 13, wobei ein Abstand zwischen benachbarten Zacken so bemessen ist, dass die Zacken im Ruhezustand um das Gehäuse herum gebogen werden können.

15. Tragbare EKG-Signalübertragungsvorrichtung nach einem der Ansprüche 5 sowie 11 bis 14, wobei die erste Unterarmelektrode zur Anbringung unter einer linken Achselhöhle des Patienten eingerichtet ist.

16. Tragbare EKG-Signalübertragungsvorrichtung nach einem der Ansprüche 1 bis 15, wobei die Elektroden mit einem Siebdruckverfahren hergestellt werden.

17. Tragbare EKG-Signalübertragungsvorrichtung nach einem derAnsprüche 1 bis 16, wobei:
die Vielzahl von Brustkorbelektroden (16) überzählige Elektroden enthält,
die EKG-Signalübertragungsschaltung einen Größenwählschalter (37, 38) mit einer Vielzahl von Einstellungen jeweils für einen unterschiedlichen Größenbereich des Patienten enthält, und
die EKG-Signalübertragungsschaltung auf eine gewählte Einstellung des Größenwählschalters anspricht und verschiedene der Brustkorbelektroden auswählt.

18. Tragbare EKG-Signalübertragungsvorrichtung nach einem der vorangehenden Ansprüche, die einen Sender (76) zum Senden eines Signals enthält, das für das EKG eines Patienten steht.

19. Tragbare EKG-Signalübertragungsvorrichtung nach Anspruch 18, wobei der Sender eine Vokalisierungseinheit zum Erzeugen eines akustischen Signals enthält, das für das EKG eines Patienten steht.

20. Tragbare EKG-Signalübertragungsvorrichtung nach Anspruch 18, wobei der Sender eine digitale Schaltung (71) zum Erzeugen eines Digitalsignals enthält, das für das EKG eines Patienten steht.

21. Tragbare EKG-Signalübertragungsvorrichtung nach Anspruch 19, die des Weiteren einen HF-Sender zum Senden eines HF-modulierten Trägersignals enthält, das für das EKG eines Patienten steht.

22. Tragbare EKG-Signalübertragungsvorrichtung nach einem der vorangehenden Ansprüche, die des Weiteren eine Befestigungseinrichtung (46) zum Befestigen der Vorrichtung um den Körper eines Patienten herum enthält.

23. Tragbare EKG-Signalübertragungsvorrichtung nach einem der Ansprüche 18 bis 21, wobei der Sender des Weiteren so eingerichtet ist, dass er Daten sendet, die wenigstens einige der folgenden Parameter anzeigen:
(a) eine Modellnummer der tragbaren EKG-Signalübertragungsvorrichtung,
(b) eine Kennungsnummer der tragbaren EKG-Signalübertragungsvorrichtung,
(c) den Zustand einer Batterie in der tragbaren EKG-Signalübertragungsvorrichtung,
(d) eine Anzeige, welche der ersten und der zweiten Elektroden ausgewählt wurde,
(e) einen ausgewählten Größenbereich des Patienten,
(f) einen Status der Befestigungseinrichtung,
(g) eine Größe des Elektrodenträgers, und
(h) einen Status des HF-Senders.

24. Tragbare EKG-Signalübertragungsvorrichtung nach einem der Ansprüche 18 bis 21, wobei der Sender so eingerichtet ist, dass er sendet:
(a) einen Ton, der die EKG-Signalübertragungsvorrichtung identifiziert,
(b) digitale Daten, die eine Hardware-Konfiguration der EKG-Signalübertragungsvorrichtung und vorprogrammierte Daten identifizieren,
(c) den Rhythmusstreifen des Patienten, der kontinuierlich auf einem ersten Ausgangskanal gesendet wird,
(d) EKG-Daten, die von den Arm- und Beinelektroden abgeleitet und simultan auf einem zweiten und einem dritten Kanal gesendet werden,
(e) aufeinanderfolgende Paare von EKG-Daten, die von den Brustkorbelektroden abgeleitet und simultan auf dem zweiten und dem dritten Kanal gesendet werden.

25. Tragbare EKG-Signalübertragungsvorrichtung nach einem der Ansprüche 1 bis 24, die eine Einheit mit einer Panikalarmvorrichtung bildet.

## Revendications

1. Un dispositif de signalisation d'E.C.G. portatif (12), comprenant :
un boîtier (15) portant une pluralité d'électrodes thoraciques (16, 18) et destiné à être connecté à au moins une électrode d'extrémités (17, 19) à poser en différentes parties du corps d'un patient (10) de manière à mesurer le tracé de rythme du patient et un E.C.G. à 12 dérivations, et
un circuit de signalisation d'E.C.G. (65) à l'intérieur du boîtier destiné à collecter et transmettre des données fractionnelles d'E.C.G. sur au moins deux canaux de sortie en parallèle, autorisant de cette façon de transmettre les données de l'E.C.G. à 12 dérivations en moins de temps que cela pourrait être fait en collectant et transmettant les données de l'E.C.G. à 12 dérivations en série sur un seul canal de sortie en temps réel ;
**caractérisé en ce que** :
lors du fonctionnement du dispositif, le circuit de signalisation d'E.C.G. transmet sur un premier canal de sortie le tracé de rythme mesurée avec deux des électrodes, et transmet sur au moins les deuxième et troisième canaux de sortie des échantillons ou fonctions respectifs du tracé de rythme mesuré par certaines électrodes prédéterminées parmi les électrodes thoraciques et d'extrémités.

2. Dispositif de signalisation d'E.C.G. portatif selon la revendication 1, dans lequel chaque fréquence de canal module un signal d'E.C.G. fourni au canal autour d'une fréquence centrale respective choisie afin d'optimiser l'utilisation d'un spectre de fréquences disponibles.

3. Dispositif de signalisation d'E.C.G. portatif selon la revendication 2, dans lequel les fréquences centrales respectives sont approximativement 1275 Hz, 1875 Hz et 2850 Hz.

4. Dispositif de signalisation d'E.C.G. portatif selon l'une quelconque des revendications 1 à 3, dans lequel une séquence complète de transmission dure approximativement 16 secondes.

5. Dispositif de signalisation d'E.C.G. portatif selon l'une quelconque des revendications précédentes, dans lequel la au moins une électrode d'extrémités comprend des première et deuxième électrodes d'aisselle et une électrode de jambe pouvant être connectées au circuit de signalisation d'E.C.G.

6. Dispositif de signalisation d'E.C.G. portatif selon la revendication 5, dans lequel les électrodes thoraciques (16) sont disposées sur un support d'électrode (36) fin, flexible avec un rapport d'espacement et fonctionnent en combinaison avec les première et deuxième électrodes d'aisselle et l'électrode de jambe pour produire un électrocardiogramme à 12 dérivations.

7. Dispositif de signalisation d'E.C.G. portatif selon la revendication 6, dans lequel le support d'électrode flexible est pliable en un ensemble compact en cas de non-utilisation.

8. Dispositif de signalisation d'E.C.G. portatif selon l'une quelconque des revendications 1 à 7, comprenant en outre :
au moins un commutateur de sélection (30, 31) accessible depuis une surface externe du boîtier et connecté au circuit de signalisation d'E.C.G. et aux paires respectives des première et deuxième électrodes déplacées pour connecter une des premières électrodes et une correspondant à une des deuxièmes électrodes de chaque paire au circuit de signalisation d'E.C.G. selon que le patient est du sexe masculin ou féminin.

9. Dispositif de signalisation d'E.C.G. portatif selon la revendication 8, dans lequel le au moins un commutateur de sélection est constitué par des premier et second commutateurs de sélection (30, 31) déplacés pour une utilisation avec des patients respectivement de sexe masculin ou féminin.

10. Dispositif de signalisation d'E.C.G. portatif selon la revendication 9, dans lequel les premier et second commutateurs de sélection sont codés en couleur.

11. Dispositif de signalisation d'E.C.G. portatif selon la revendication 5, dans lequel :
les électrodes thoraciques (16) sont disposées sur une première surface (35) du support d'électrodes, et
la première électrode d'aisselle (18) est disposée sur une deuxième surface (40) du support d'électrodes opposée à la première surface de celui-ci.

12. Dispositif de signalisation d'E.C.G. portatif selon la revendication 11, dans lequel la première électrode d'aisselle (18) s'étend sur une certaine longueur du support d'électrodes de manière à s'adapter aux patients de différentes tailles.

13. Dispositif de signalisation d'E.C.G. portatif selon la revendication 12, dans lequel la première électrode d'aisselle (18) comprend une pluralité de dents (42) espacées l'une de l'autre et connectées à une électrode porteuse commune (43).

14. Dispositif de signalisation d'E.C.G. portatif selon la revendication 13, dans lequel un espace entre des dents adjacentes est dimensionné afin de permettre aux dents d'être pliées autour du boîtier en cas de non-utilisation.

15. Dispositif de signalisation d'E.C.G. portatif selon l'une quelconque des revendications 5 et 11 à 14, dans lequel la première électrode d'aisselle est adaptée pour être placée sous l'aisselle gauche d'un patient.

16. Dispositif de signalisation d'E.C.G. portatif selon l'une quelconque des revendications 1 à 15, dans lequel les électrodes sont formées par une technique d'impression sérigraphique.

17. Dispositif de signalisation d'E.C.G. portatif selon l'une quelconque des revendications 1 à 16, dans lequel :
la pluralité d'électrodes thoraciques (16) comprend des électrodes redondantes,
le circuit de signalisation d'E.C.G. comprend un commutateur de sélection de taille (37, 38) ayant une pluralité de réglages chacun concernant une plage différente de tailles de patient, et
le circuit de signalisation d'E.C.G. est sensible à un réglage sélectionné du commutateur de sélection de taille pour sélectionner différentes électrodes parmi les électrodes thoraciques.

18. Dispositif de signalisation d'E.C.G. portatif selon l'une quelconque des revendications précédentes, comprenant un émetteur (76) destiné à émettre un signal représentatif d'un E.C.G. de patient.

19. Dispositif de signalisation d'E.C.G. portatif selon la revendication 18, dans lequel l'émetteur comprend une unité de vocalisation destinée à produire un signal acoustique représentatif d'un E.C.G. de patient.

20. Dispositif de signalisation d'E.C.G. portatif selon la revendication 18, dans lequel l'émetteur comprend un circuit numérique (71) destiné à produire un signal numérique représentatif d'un E.C.G. de patient.

21. Dispositif de signalisation d'E.C.G. portatif selon la revendication 19, comprenant en outre un émetteur RF destiné à transmettre un signal RF porteur modulé représentatif d'un E.C.G. de patient.

22. Dispositif de signalisation d'E.C.G. portatif selon l'une quelconque des revendications précédentes, comprenant en outre une attache (46) destinée à immobiliser le dispositif autour du corps du patient.

23. Dispositif de signalisation d'E.C.G. portatif selon l'une quelconque des revendications 18 à 21, dans lequel l'émetteur est en outre adapté pour émettre des données indiquant au moins certains des paramètres suivants :
(a) un numéro de modèle du dispositif de signalisation d'E.C.G. portatif,
(b) un numéro d'identification (ID) du dispositif de signalisation d'E.C.G. portatif,
(c) l'état d'une pile à l'intérieur du dispositif de signalisation d'E.C.G. portatif,
(d) une indication de laquelle des première et deuxième électrodes était sélectionnée,
(e) la plage sélectionnée pour la taille du patient,
(f) l'état de l'attache,
(g) la taille du support d'électrodes, et
(h) l'état de l'émetteur RF.

24. Dispositif de signalisation d'E.C.G. portatif selon l'une quelconque des revendications 18 à 21, dans lequel l'émetteur est adapté afin d'émettre :
(a) une tonalité identifiant le dispositif de signalisation d'E.C.G.,
(b) des données numériques identifiant une configuration matérielle du dispositif de signalisation d'E.C.G. et des données pré-programmées,
(c) le tracé de rythme du patient étant émis continuellement sur un premier canal de sortie,
(d) des données d'E.C.G. dérivées des électrodes d'aisselle et de jambe étant émises simultanément sur des deuxième et troisième canaux,
(e) des paires successives de données d'E.C.G. dérivées des électrodes thoraciques étant émises simultanément sur les deuxième et troisième canaux.

25. Dispositif de signalisation d'E.C.G. portatif selon l'une quelconque des revendications 1 à 24, étant intégré avec un dispositif d'alarme de panique.
